(19) Europäisches Patentamt / European Patent Office / Office européen des brevets

(11) **EP 4 356 826 A1**

(12) **EUROPEAN PATENT APPLICATION**
published in accordance with Art. 153(4) EPC

(43) Date of publication:
**24.04.2024  Bulletin 2024/17**

(21) Application number: **22825069.2**

(22) Date of filing: **16.06.2022**

(51) International Patent Classification (IPC):
***A61B 5/021*** *(2006.01)*

(52) Cooperative Patent Classification (CPC):
**A61B 5/021**

(86) International application number:
**PCT/JP2022/024216**

(87) International publication number:
**WO 2022/265081 (22.12.2022 Gazette 2022/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.06.2021  JP 2021101120**

(71) Applicant: **TERUMO Kabushiki Kaisha**
**Tokyo 151-0072 (JP)**

(72) Inventors:
• **HATTA Tomonori**
**Ashigarakami-gun, Kanagawa 259-0151 (JP)**

• **ICHIKAWA Ryo**
**Ashigarakami-gun, Kanagawa 259-0151 (JP)**
• **NARITA Makoto**
**Ashigarakami-gun, Kanagawa 259-0151 (JP)**
• **UTSUGIDA Tomoki**
**Ashigarakami-gun, Kanagawa 259-0151 (JP)**
• **EZASHI Yoshiharu**
**Ashigarakami-gun, Kanagawa 259-0151 (JP)**

(74) Representative: **Casalonga**
**Casalonga & Partners**
**Bayerstraße 71/73**
**80335 München (DE)**

(54) **VENTRICULAR PRESSURE WAVEFORM ESTIMATION DEVICE, VENTRICULAR PRESSURE WAVEFORM ESTIMATION METHOD, VENTRICULAR PRESSURE WAVEFORM ESTIMATION PROGRAM, AND ?ULMONARY ARTERY PRESSURE ?AVEFORM ESTIMATION DEVICE**

(57) A ventricular pressure waveform estimation device 10 includes a calculation unit 12. The calculation unit 12 estimates each of a plurality of coefficients of a model equation 17 including the coefficients and indicating a left ventricular pressure waveform or a right ventricular pressure waveform by using values of one or more parameters related to a heartbeat or an arterial pressure to estimate at least a part of the left ventricular pressure waveform or the right ventricular pressure waveform.

FIG. 1

EP 4 356 826 A1

**Description**

Technical Field

The present disclosure relates to a ventricular pressure waveform estimation device, a ventricular pressure waveform estimation method, a ventricular pressure waveform estimation program, and a pulmonary artery pressure waveform estimation device.

Background Art

It is important to measure pressure in a ventricle in order to grasp a disease state related to the heart and to assess a cardiac function. For example, patients with heart failure can require hospitalization and treatment due to acute exacerbation. In addition, congestion accompanying exacerbation may cause symptoms such as accumulation of water in lungs and difficulty in breathing. An increase in ventricular pressure is known as a sign of the accumulation of water in the lungs. It is desirable to grasp signs of exacerbation at an early stage and suppress symptoms accompanying the congestion by medication or the like. Left ventricular end-diastolic pressure (LVEDP), pulmonary capillary wedge pressure (PCWP), and pulmonary artery pressure (PAP) are known as indices indicating an omen of exacerbation at an early stage. The LVEDP refers to the left ventricular end-diastolic pressure. The PCWP refers to pulmonary capillary wedge pressure. The pulmonary capillary wedge pressure is also referred to as pulmonary arterial wedge pressure or "PAWP" for short, pulmonary wedge pressure or "PWP" for short, or pulmonary artery occlusion pressure or "PAOP" for short. The PAP refers to the pulmonary artery pressure.

In general, methods of using pigtail catheters or balloon catheters are used to measure left ventricular pressure, right ventricular pressure, and pulmonary artery pressure. However, these methods are invasive methods in which a catheter is inserted from an artery or a vein at a base of a foot, an artery of an arm, or the like into the heart or to the vicinity of the heart, and a burden on a patient's body is great. It is desirable to obtain an estimation of a ventricular pressure waveform in the heart and a pulmonary artery pressure waveform by a simple method and in a non-invasive or minimally invasive manner so as not to give a burden on the patient's body. Patent Literature 1 discloses a technique of estimating an arterial pressure waveform from a heart sound, upper arm cuff pressure, and K-sounds to calculate LVEDP. "K" is an abbreviation for Korotkoff.

Citation List

Patent Literature

Patent Literature 1: US 2015/0265163 A

Summary of Invention

Technical Problem

Clinically of heart failure, information on waveform shapes of left ventricular pressure and right ventricular pressure and information on a waveform shape of pulmonary artery pressure including information of contractility of a left heart and a right heart are important clinically for heart failure. Conventionally, the waveform shapes of the left and right ventricular pressures and the pulmonary artery pressure have been measured by invasive methods.

In Patent Literature 1, in order to estimate pressure in a left heart, a cuff is wound around an upper arm, and a time interval from a Q-wave to a K-sound of an electrocardiogram waveform is measured while slowly lowering the cuff pressure. Further, a curve, such as a polynomial curve or a cosine curve, is made to fit between the measured time interval and the cuff pressure. In Patent Literature 1, the LVEDP is estimated using this curve. However, this curve is an estimate of an arterial pressure waveform in the periphery and does not accurately reflect the left ventricular pressure waveform.

Therefore, an object of the present disclosure, which has been made in light of such points, is to estimate a waveform shape of left ventricular pressure, right ventricular pressure and/or pulmonary artery pressure by a non-invasive or minimally invasive method.

Solution to Problem

(1) A ventricular pressure waveform estimation device according to an aspect of the present disclosure includes a calculation unit configured to estimate a plurality of coefficients of a model equation, the model equation configured to indicate including the coefficients and indicating a left ventricular pressure waveform or a right ventricular pressure waveform by using values of one or more parameters related to a heartbeat or an arterial pressure to estimate at least a part of the left ventricular pressure waveform or the right ventricular pressure waveform.

(2) As an embodiment, in the ventricular pressure waveform estimation device according to (1), the calculation unit is configured to estimate each coefficient of the plurality of coefficients of the model equation by using the one or more parameters as explanatory variables of a machine learning model.

(3) As an embodiment, in the ventricular pressure waveform estimation device according to (2), the machine learning model is constructed by acquiring a measured waveform of at least a part of the left ventricular pressure waveform or the right ventricular pressure waveform, calculating values of the plurality of coefficients by fitting the model equation to the measured waveform, generating a data set for machine learning in which at least any of the one or more parameters in the acquisition of the measured waveform are used as explanatory variables and the plurality of coefficients are used as objective variables, respectively, and executing machine learning using the data set for machine learning.

(4) As an embodiment, in the ventricular pressure waveform estimation device according to any one of (1) to (3), the one or more parameters include one or more parameters related to the heartbeat and one or more parameters related to the arterial pressure.

(5) As an embodiment, in the ventricular pressure waveform estimation device according to any one of (1) to (4), the model equation is

[Math. 1]

$$P(t) = \frac{a}{1 + be^{-kt}} + c \qquad (1)$$

where a, b, c, and k are the coefficients.

(6) As an embodiment, in the ventricular pressure waveform estimation device according to any one of (1) to (5), the one or more parameters include at least any of a diastolic blood pressure, a systolic blood pressure, a maximum velocity of a pulse pressure waveform rise, a blood pressure value difference between a rise start point of a peripheral pulse pressure waveform and a dicrotic notch, a pulse wave augmentation index pulse wave augmentation index, a heart rate, isovolumetric systolic time, a pulse wave velocity, and systolic time.

(7) As an embodiment, in the ventricular pressure waveform estimation device according to any one of (1) to (6), the calculation unit is configured to calculate a value of left ventricular end-diastolic pressure (LVEDP) based on at least a part of the estimated left ventricular pressure waveform.

(8) As an embodiment, in the ventricular pressure waveform estimation device according to any one of (1) to (6), the calculation unit is configured to calculate a value of pulmonary capillary wedge pressure (PCWP) based on at least a part of the estimated right ventricular pressure waveform.

(9) As an embodiment, in the ventricular pressure waveform estimation device according to any one of (1) to (8), the calculation unit is configured to calculate an index of contractility of a left ventricle or a right ventricle based on a slope of the estimated left ventricular pressure waveform or the estimated right ventricular pressure waveform.

(10) As an embodiment, the ventricular pressure waveform estimation device according to any one of (1) to (9) further including: a first detection unit configured to detect one or more first biological signals related to the heartbeat; and one or more second detection units configured to detect one or more second biological signals related to the arterial pressure, in which the values of the one or more parameters is determined based on one or more of the first biological signal and the one or more second biological signal.

(11) As one embodiment, in the ventricular pressure waveform estimation device according to (10), the first detection unit and the second detection unit are configured to detect the first biological signal and the second biological signal, respectively, by a non-invasive method.

(12) As one embodiment, in the ventricular pressure waveform estimation device according to (10), the first detection unit is configured to detect the one or more first biological signals by a non-invasive method or by a minimally invasive method, and the one more second detection units are configured to detect the one or more second biological signals by a non-invasive method or by a minimally invasive method.

(13) A ventricular pressure waveform estimation method as one aspect of the present disclosure is a method for estimating at least a part of a left ventricular pressure waveform or a right ventricular pressure waveform by a computer, the method including estimating each of a plurality of coefficients of a model equation including the coefficients and indicating a left ventricular pressure waveform or a right ventricular pressure waveform by using values of one or more parameters related to a heartbeat or an arterial pressure to estimate at least a part of the left ventricular pressure waveform or the right ventricular pressure waveform.

(14) A ventricular pressure waveform estimation program according to an aspect of the present disclosure causes a computer to execute processing of estimating each of a plurality of coefficients of a model equation including the coefficients and indicating a left ventricular pressure waveform or a right ventricular pressure waveform by using values of one or more parameters related to a heartbeat or an arterial pressure to estimate at least a part of the left ventricular pressure waveform or the right ventricular pressure waveform.

(15) A pulmonary artery pressure waveform estimation device according to an aspect of the present disclosure includes a calculation unit configured to estimate each of a plurality of coefficients of a model equation including the coefficients and indicating a pulmonary artery pressure waveform by using values of one or more parameters related to a heartbeat or an arterial pressure to estimate at least a part of the pulmonary artery pressure waveform.

(16) As one embodiment, in the pulmonary artery pressure waveform estimation device according to (15), the calculation unit is configured to estimate each coefficient of the plurality of coefficients of the model equation by using the one or more parameters as explanatory variables of a machine learning model.

(17) As one embodiment, in the pulmonary artery pressure waveform estimation device according to (16), the machine learning model is constructed by acquiring a measured waveform of at least a part of the pulmonary artery pressure waveform, calculating values of the plurality of coefficients by fitting the model equation to the measured waveform, generating a data set for machine learning in which the one or more parameters in the acquisition of the measured waveform are used as explanatory variables and the plurality of coefficients are used as objective variables, respectively, and executing machine learning using the data set for machine learning.

(18) As an embodiment, in the pulmonary artery pressure waveform estimation device according to any one of (15) to (17), the one or more parameters include one or more parameters related to a heartbeat and one or more parameters related to arterial pressure.

(19) As an embodiment, in the pulmonary artery pressure waveform estimation device according to any one of (15) to (18), the model equation is

[Math. 2]

$$P(t) = \frac{a}{1 + be^{-kt}} + c$$

where a, b, c, and k are the coefficients.

(20) As an embodiment, in the pulmonary artery pressure waveform estimation device according to any one of (15) to (19), the one or more parameters include at least any of a diastolic blood pressure, a systolic blood pressure, a maximum velocity of a pulse pressure waveform rise, a blood pressure value difference between a rise start point of a peripheral pulse pressure waveform and a dicrotic notch, a pulse wave augmentation index pulse wave augmentation index, a heart rate, isovolumetric systolic time, a pulse wave velocity, and systolic time.

(21) As an embodiment, in the pulmonary artery pressure waveform estimation device according to any one of (15) to (20), the calculation unit is configured to calculate a value of pulmonary artery pressure based on at least a part of the estimated pulmonary artery pressure waveform.

(22) As an embodiment, in the pulmonary artery pressure waveform estimation device according to any one of (15) to (20), the calculation unit is configured to estimate a value of left ventricular end-diastolic pressure (LVEDP) based on at least a part of the estimated pulmonary artery pressure waveform.

(23) As an embodiment, in the pulmonary artery pressure waveform estimation device according to any one of (15) to (20), the calculation unit is configured to calculate a value of pulmonary capillary wedge pressure (PCWP) based on at least a part of the estimated pulmonary artery pressure waveform.

(24) As an embodiment, in the pulmonary artery pressure waveform estimation device according to any one of (15) to (23), the calculation unit is configured to calculate an index of contractility of a left ventricle or a right ventricle based on a slope of the estimated pulmonary artery pressure waveform.

(25) As an embodiment, the pulmonary artery pressure waveform estimation device according to any one of (15) to (24) further including: a first detection unit configured to detect one or more first biological signals related to a heartbeat; and one or more second detection units configured to detect one or more second biological signals related to arterial pressure, in which the value of the parameter is determined based on at least any of the first biological signal and the second biological signal.

(26) As one embodiment, in the pulmonary artery pressure waveform estimation device according to (25), the first detection unit and the second detection unit are configured to detect the first biological signal and the second biological signal, respectively, by a non-invasive method.

(27) As one embodiment, in the pulmonary artery pressure waveform estimation device according to (25), the first detection unit and the second detection unit are configured to detect the first biological signal and the second biological signal, respectively, by a non-invasive or minimally invasive method.

Advantageous Effects of Invention

According to the present disclosure, the waveform shape of the left ventricular pressure or the right ventricular pressure can be estimated by the non-invasive or minimally invasive method.

Brief Description of Drawings

Fig. 1 is a block diagram illustrating a configuration of a ventricular pressure waveform estimation device according to an embodiment of the present disclosure.
Fig. 2 is a view illustrating an example of a left ventricular pressure waveform.
Fig. 3 is a diagram illustrating an equivalent circuit representing a relationship between a cardiac output (I(t)) and aortic pressure (P(t)) in a Windkessel model.
Fig. 4 is a flowchart illustrating a procedure for constructing a machine learning model for estimating each of coefficients of a model equation.
Fig. 5 is a diagram for describing the procedure for constructing the machine learning model.
Fig. 6 is a view illustrating an example of fitting to a measured waveform based on the model equation.
Fig. 7 is a diagram for describing estimation of the coefficients of the model equation using the machine learning model.
Fig. 8 is a view illustrating examples of non-invasive parameters.
Fig. 9 is a flowchart illustrating a procedure for estimating the right ventricular pressure waveform or the left ventricular pressure waveform.
Fig. 10 is a block diagram illustrating a configuration of a pulmonary artery pressure waveform estimation device according to an embodiment of the present disclosure.
Fig. 11 is a flowchart illustrating a procedure for estimating a pulmonary artery pressure waveform.

Description of Embodiments

A ventricular pressure waveform estimation device 10 according to an embodiment of the present disclosure will be described with reference to the drawings. The ventricular pressure waveform estimation device 10 estimates at least a part of a left ventricular pressure waveform or a right ventricular pressure waveform based on one or more parameters related to the heartbeat or arterial pressure acquired by at least one of a non-invasive method and a minimally invasive method. Note that the term "non-invasive" in the present application means that no disorder is given to a human body. The term "minimally invasive" means that incision around the heart of a human body or insertion of an instrument into a ventricle of the human body using a heart catheter is not involved.

(Configuration of Ventricular Pressure Waveform Estimation Device)

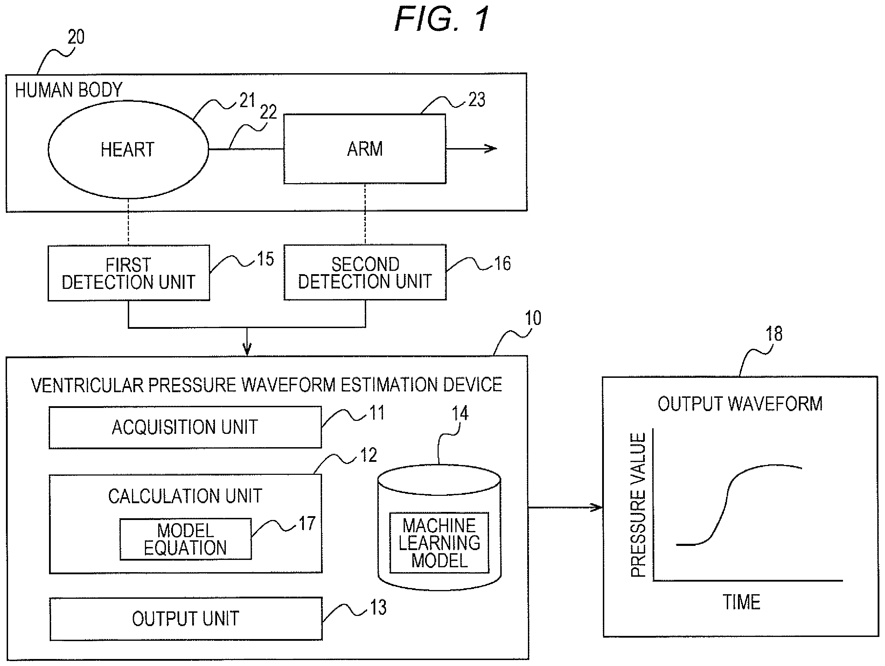

As illustrated in Fig. 1, the ventricular pressure waveform estimation device 10 includes an acquisition unit 11, a calculation unit 12, an output unit 13, and a storage unit 14. The ventricular pressure waveform estimation device 10 is a computer equipped with a program for estimating a ventricular pressure waveform. The ventricular pressure waveform estimation device 10 is, for example, dedicated equipment, general-purpose equipment such as a personal computer (PC), or server equipment belonging to a cloud computing system or another computing system.
The acquisition unit 11 is configured to be capable of acquiring values of one or more parameters related to a heartbeat or an arterial pressure. The acquisition unit 11 may receive the value of the one or more parameters as an electrical signal from the outside of the ventricular pressure waveform estimation device 10. The acquisition unit 11 may read an electronic file including the value of the one or more parameters from a storage medium. The acquisition unit 11 may include an input terminal that receives an input from the outside, an interface for communication with an external device, a reading device that reads information of the storage medium, and the like.
As illustrated in Fig. 1, the acquisition unit 11 may acquire a value of the parameter related to the heartbeat or arterial pressure from a first detection unit 15 and a second detection unit 16 that detect biological signals from a human body 20 by a non-invasive or minimally invasive method. The ventricular pressure waveform estimation device 10 may include the first detection unit 15 and the second detection unit 16. A parameter based on a biological signal acquired by the non-invasive method is referred to as a non-invasive parameter. A parameter based on a biological signal acquired by the minimally invasive method is referred to as a minimally invasive parameter.

The first detection unit 15 may detect one or more first biological signals related to the heartbeat of a heart 21 only by a non-invasive method. The first detection unit 15 may include, for example, at least any of an electrocardiogram (ECG) that detects a heartbeat, an optical sensor, an impedance sensor, and an acceleration sensor. The first detection unit 15 may include, for example, at least one of a microphone that detects a heart sound, an acceleration sensor, a vibration sensor, and a piezoelectric sensor.

The second detection unit 16 may detect one or more second biological signals related to the arterial pressure at a site away from the heart 21, such as an arm 23, for example, only by a non-invasive method. The second detection unit 16 can use, for example, a cuff pressure sensor, photoplethysmography (PPG), remote photoplethysmography (rPPG), tonometry, and the like. The measurement of pulse pressure using the cuff pressure sensor includes a method using a K-sound and an oscillometric method. In this case, the second detection unit 16 can detect the arterial pressure of a site including at least one of a carotid artery, a brachial artery, a radial artery, a fingertip, and an ear.

In a case where the second detection unit 16 detects the arterial pressure by a minimally invasive method, it is possible to use an invasive blood pressure measurement method such as a method of, for example, a contrast catheter, an arterial line (A-line), an intravascular implant (a stent, an artificial blood vessel, or an implantation sensor), subcutaneous implantation, or the like. In this case, the second detection unit 16 can detect the arterial pressure of the site including at least one of the carotid artery, the brachial artery, and the radial artery.

The first detection unit 15 may calculate a value of the parameter related to the heartbeat from the first biological signal. The second detection unit 16 may calculate a value of the parameter related to the arterial pressure from the second biological signal. The parameter related to the heartbeat and the parameter related to the arterial pressure may be calculated in the ventricular pressure waveform estimation device 10 from the first biological signal and the second biological signal, instead of the first detection unit 15 and the second detection unit 16. For example, the calculation unit 12 of the ventricular pressure waveform estimation device 10 may calculate a value of the parameter related to the heartbeat or arterial pressure by combining the first biological signal and the second biological signal.

The calculation unit 12 includes at least one processor, and executes various types of calculation processing required in the ventricular pressure waveform estimation device 10 while controlling each unit of the ventricular pressure waveform estimation device 10. The processor includes a general-purpose processor such as a central processing unit (CPU) or a dedicated processor specialized for specific processing. The calculation unit 12 may include an application specific integrated circuit (ASIC), a digital signal processor (DSP), a programmable logic device (PLD), a field-programmable gate array (FPGA), or any combination thereof. The calculation unit 12 may include a memory constructed in the processor or a memory independent of the processor.

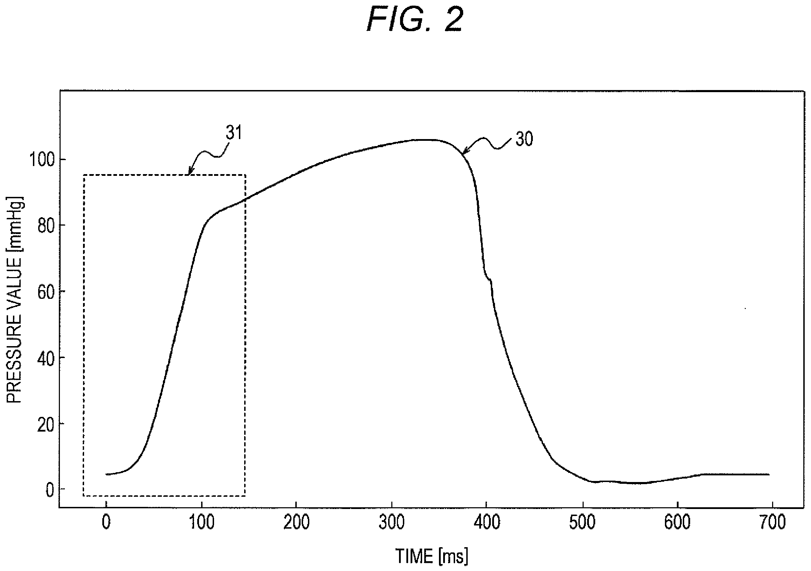

The calculation unit 12 estimates at least a part of the left ventricular pressure waveform or the right ventricular pressure waveform using the value of the one or more parameters related to the heartbeat or arterial pressure acquired by the acquisition unit 11. For example, FIG 2 illustrates a left ventricular pressure waveform 30 corresponding to one heartbeat. The calculation unit 12 estimates a partial waveform 31 that is a part of the left ventricular pressure waveform 30 surrounded by a dashed line. The partial waveform 31 is a waveform in systole of a left ventricle. An estimation target is, for example, a period of about 150 milliseconds (ms) to 200 milliseconds (ms) after the left ventricle starts to contract.

The calculation unit 12 has a model equation indicating the left ventricular pressure waveform or the right ventricular pressure waveform. This model equation is expressed by the following Formula (1).

[Math. 3]

$$P(t) = \frac{a}{1 + be^{-kt}} + c \qquad (1)$$

The model equation includes four coefficients a, b, c, and k. The calculation unit 12 estimates at least a part of the left ventricular pressure waveform or the right ventricular pressure waveform by estimating the respective coefficients a, b, c, and k of the model equation. A constructed machine learning model is used to estimate each of the coefficients. An estimation method of the left ventricular pressure waveform or the right ventricular pressure waveform executed by the calculation unit 12 will be described in more detail below.

The output unit 13 includes at least one output interface. The output unit 13 outputs data of an output waveform 18 indicating a time change in the internal pressure of the left ventricle or a right ventricle obtained by an operation of the ventricular pressure waveform estimation device 10. The output unit 13 may output the data of the output waveform 18 to be processed by a device in a subsequent stage. Instead of outputting the output waveform 18 to another device, the ventricular pressure waveform estimation device 10 may further perform processing in its own device based on this waveform data.

The storage unit 14 includes at least one semiconductor memory, at least one magnetic memory, at least one optical memory, or any combination thereof. The semiconductor memory is, for example, a random access memory (RAM) or a read only memory (ROM). The RAM is, for example, a static random access memory (SRAM) or a dynamic random access memory (DRAM). The ROM is, for example, an electrically erasable programmable read only memory (EEPROM). The storage unit 14 functions as, for example, a main storage device, an auxiliary storage device, or a cache memory. The storage unit 14 stores data used for the operation of the ventricular pressure waveform estimation device 10, and data obtained by the operation of the ventricular pressure waveform estimation device 10. The storage unit 14 stores a machine learning model to be used to estimate the ventricular pressure waveform.

Functions of the ventricular pressure waveform estimation device 10 are implemented by executing a ventricular pressure waveform estimation program (hereinafter, referred to as the "program") according to the present embodiment by a processor serving as the calculation unit 12. That is, the functions of the ventricular pressure waveform estimation device 10 are implemented by software. The program causes a computer to function as the ventricular pressure waveform estimation device 10 by causing the computer to execute the operation of the ventricular pressure waveform estimation device 10. That is, the computer functions as the ventricular pressure waveform estimation device 10 by executing the operation of the ventricular pressure waveform estimation device 10 according to the program.

The program can be stored in a non-transitory computer-readable medium. The non-transitory computer-readable medium is, for example, a flash memory, a magnetic recording device, an optical disc, a magneto-optical recording medium, or a ROM. The program is distributed by, for example, selling, transferring, or lending a portable medium such as a secure digital (SD) card, a digital versatile disc (DVD), or a compact disc read only memory (CD-ROM) storing the program. The program may be distributed by storing the program in a storage of a server and transferring the program from the server to another computer. The program may be provided as a program product.

The computer temporarily stores, for example, the program stored in the portable medium or the program transferred from the server in the main storage device. Then, the computer reads the program stored in the main storage device by the processor, and executes processing according to the read program by the processor. The computer may read the program directly from the portable medium and execute the processing according to the program. The computer may sequentially execute the processing according to the received program each time the program is transferred from the server to the computer. The processing may be executed by a so-called application service provider (ASP) type service that implements functions only by an execution instruction and result acquisition without transferring the program from the server to the computer. The program includes information that is provided for processing by an electronic computer and is equivalent to the program. For example, data that is not a direct command to the computer but has a property that defines processing of the computer corresponds to the "information equivalent to the program".

Some or all of the functions of the ventricular pressure waveform estimation device 10 may be implemented by a programmable circuit or a dedicated circuit as the calculation unit 12. That is, some or all of the functions of the ventricular pressure waveform estimation device 10 may be implemented by hardware.

(Derivation of Model Equation)

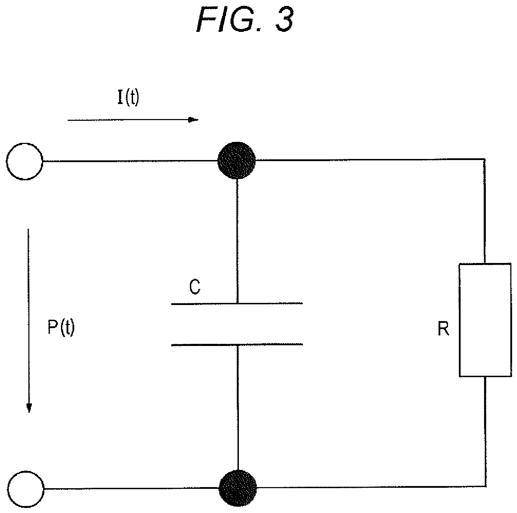

Hereinafter, a method for deriving the model equation will be described. A Windkessel model is known as a model for describing a blood pressure waveform in an artery. According to a two-element model of the Windkessel model, a fluctuation in a blood pressure pulse wave can be replaced and expressed by an equivalent electric circuit illustrated in Fig. 3 in which a capacitance C of a capacitor corresponds to arterial compliance and a resistance R of a resistor corresponds to a peripheral vascular resistance of systemic circulation. In this model, the volume of blood pumped out of the heart is expressed as a current I(t). In addition, a blood pressure of the aorta is expressed as a potential P(t). The current I(t) and the potential P(t) are functions of time t.

At this time, a relationship between the current I(t) and the potential P(t) is expressed by a differential equation illustrated in the following Formula (2).

[Math. 4]

$$I(t) = \frac{P(t)}{R} + C\frac{dP(t)}{dt} \qquad (2)$$

Formula (2) can be solved for the potential P(t) representing the blood pressure of the aorta assuming that the current I(t) representing a blood flow is a sigmoid function. The sigmoid function is generally used as a model of properties possessed by biological nerve cells. Myocardial contraction is caused by an action potential with respect to a cardiomyocyte, and thus, can be expressed in the same manner as an action potential of a nerve in principle. Therefore, I(t) in Formula (2) can be placed as the sigmoid function. In addition, it is possible to assume that I(t) follows the sigmoid function even from experimental data.

If the differential equation of Formula (2) is solved with I(t) as the sigmoid function of

[Math. 5]

$$I(t) = \frac{I_0}{1 + e^{-kt}} \qquad (3)$$

P(t) is obtained as the following formula.
[Math. 6]

$$P(t) = \frac{RI_0}{1 + (1 - RCk)e^{-kt}} + c_1 e^{-t/RC} \qquad (4)$$

Here, $I_0$ denotes an amplitude. Further, $c_1$ and k denote arbitrary coefficients.
Formula (1) of the above-described model equation is obtained by rewriting Formula (4). Formula (1) is reexpressed as follows.
[Math. 7]

$$P(t) = \frac{a}{1 + be^{-kt}} + c \qquad (1)$$

Coefficients a, b, c, and k in Formula (1) denote arbitrary coefficients. Note that the second term of Formula (4) is $RC \approx 1$, and thus, can be assumed to be substantially constant in the range of t = 0 to 200 ms (0 to 0.2 s).
The aorta is a blood vessel through which blood pumped out of the left ventricle passes first, and thus, the blood pressure of the aorta becomes close to the internal pressure of the left ventricle. Therefore, Formula (1) can be re-read as one indicating the left ventricular pressure waveform. In addition, when the blood pressure of a pulmonary artery is replaced by the potential P(t) and the volume of blood pumped out of the right ventricle is replaced by I(t), the right ventricular pressure waveform can also be expressed by Formula (1) with the similar model.

(Method for Constructing Machine Learning Model)

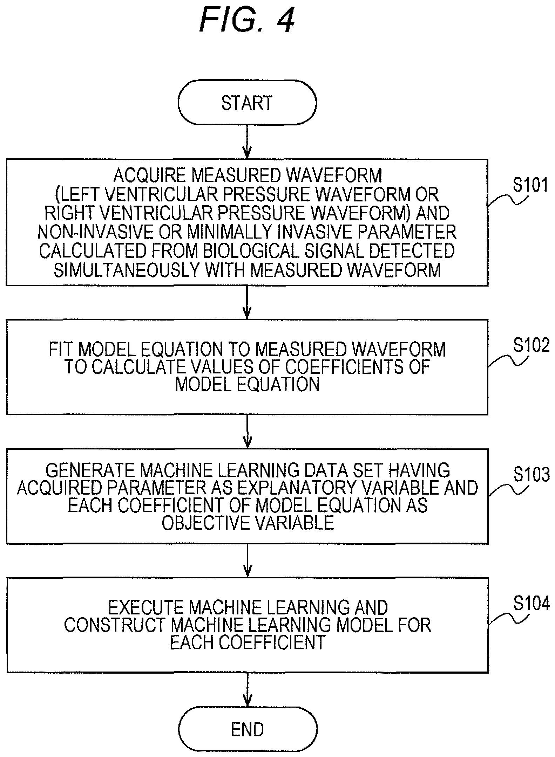

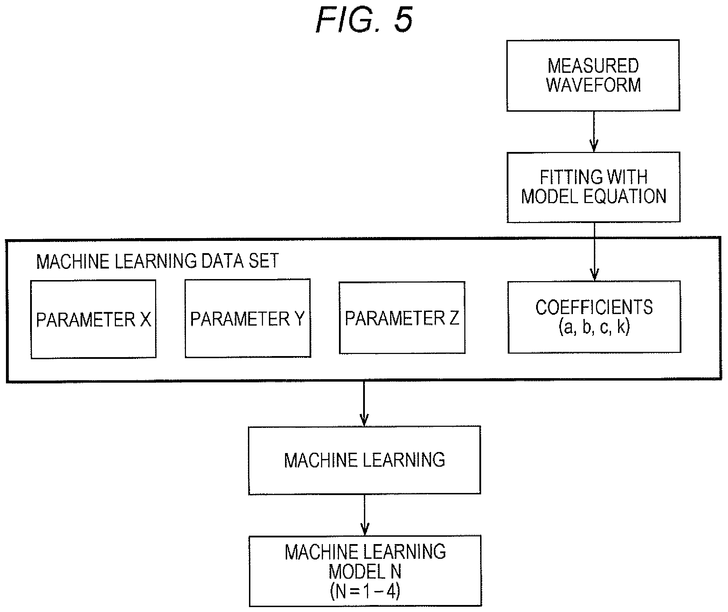

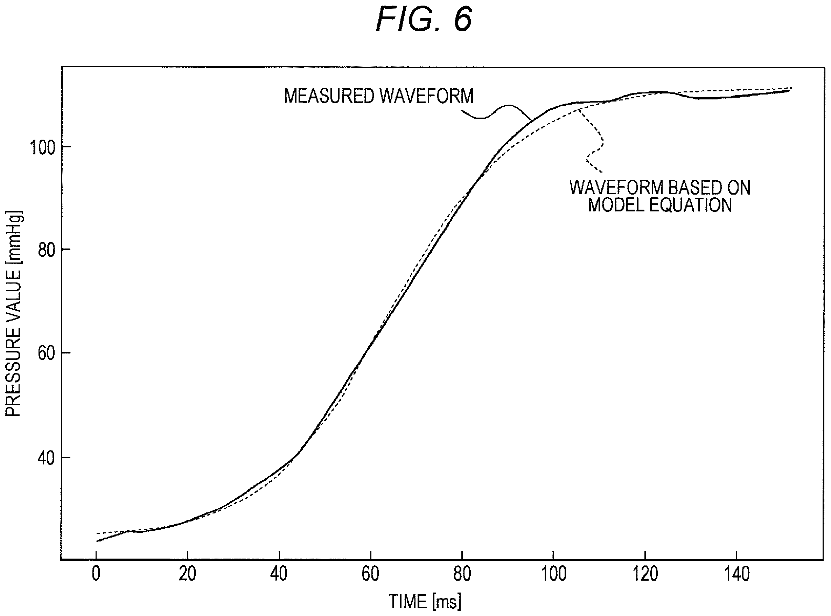

The ventricular pressure waveform estimation device 10 stores a machine learning model constructed in advance in the storage unit 14. The machine learning model may be constructed using a computer for machine learning different from the ventricular pressure waveform estimation device 10. A procedure for constructing the machine learning model will be described with reference to a flowchart of Fig. 4 and Fig. 5.
First, a measured waveform of the left ventricular pressure or the right ventricular pressure and at least any of a non-invasive parameter and a minimally invasive parameter related to the heartbeat or arterial pressure calculated from a biological signal detected simultaneously with the measured waveform are acquired (step S101). The non-invasive parameter and the minimally invasive parameter will be referred to simply as parameters hereinafter. In Fig. 5, a plurality of parameters are illustrated as parameters X, Y, and Z. The plurality of parameters can include at least one parameter related to the heartbeat and at least one parameter related to the arterial pressure. The measured waveform of the left ventricular pressure or the right ventricular pressure may be measured by an invasive method using, for example, a pigtail catheter, a balloon catheter, or the like. It is desirable to acquire a large number of combinations of the measured waveforms and parameters. As the measured waveforms and parameters, for example, pieces of data measured in a normal state and in an abnormal state of a large number of patients with heart failure can be used. The pieces of data of the measured waveforms and parameters may be collected by a technician or the like from measured data accumulated in a medical institution or the like and input to the computer for machine learning.
The computer for machine learning calculates values of the coefficients a, b, c, and k of the model equation by fitting the model equation (equation (1)) to each of the measured waveforms for the measured waveforms (step S102). A nonlinear least squares method can be used for the fitting of the model equation. The nonlinear least squares method includes a steepest descent method, the Gauss-Newton method, the Levenberg-Marquardt method, the Powell's least square method, and the like. As illustrated in Fig. 6, the coefficients of the model equation are determined such that a waveform of the model equation is closest to a measured waveform. The measured waveform in Fig. 6 corresponds to, for example, the partial waveform 31 of the left ventricular pressure waveform 30 illustrated in Fig. 2.
The computer for machine learning generates a data set for machine learning having the parameters acquired in step S101 as explanatory variables and the coefficients a, b, c, and k of the model equation calculated in step S102 as objective variables (step S103). As the data set for machine learning, a plurality of data sets obtained by collecting data for each attribute may be generated in consideration of attributes such as an age group and gender of the patients.

The computer for machine learning executes machine learning using the data set for machine learning generated in step S103, and constructs a machine learning model for each of the coefficients (step S104). The constructed machine learning model can be rephrased as a learned model. If the plurality of data sets are generated for each of the attributes of the patients, a plurality of the machine learning models may be constructed for each of the coefficients in accordance with the attribute of the patients. The machine learning models are constructed for the coefficients a, b, c, and k, respectively. Learning methods of the machine learning include a gradient boosting method, a nonlinear multiple regression method, and a neural network.

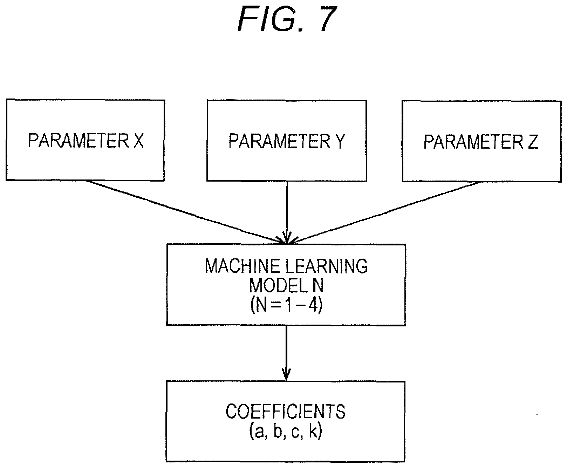

The machine learning models constructed in advance as described above are stored in the storage unit 14 of the ventricular pressure waveform estimation device 10. Therefore, the ventricular pressure waveform estimation device 10 has four machine learning models N (N = 1 to 4) corresponding to the four coefficients a, b, c, and k of the model equation, respectively, as illustrated in Fig. 7. The machine learning model N (N = 1 to 4) means an Nth machine learning model among the four machine learning models. The ventricular pressure waveform estimation device 10 estimates each of the four coefficients a, b, c, and k using values of the plurality of parameters X, Y, and Z as inputs. The plurality of parameters X, Y, and Z are non-invasive parameters or minimally invasive parameters. The number of parameters of the parameters X, Y, and Z is not limited to three. The number of parameters of the plurality of parameters X, Y, and Z can be set to two or four or more. A combination of the plurality of parameters X, Y, and Z may be different for each machine learning model (1 - N) for the four coefficients a, b, c, and k. As the plurality of parameters X, Y, and Z, a highly relevant parameter may be selected for each of the coefficients a, b, c, and k in step S104.

(Example of Non-Invasive Parameter)

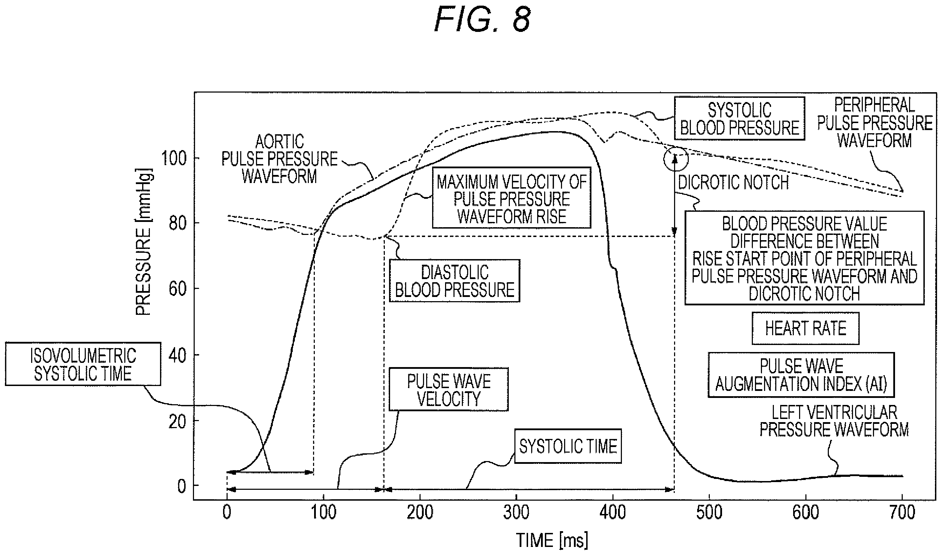

If parameters to be used in estimating the left ventricular pressure waveform or the right ventricular pressure waveform are only non-invasive parameters detected by a non-invasive method, a burden on a patient's body is relatively small. FIG 8 illustrates examples of non-invasive parameters used in the ventricular pressure waveform estimation device 10. Fig. 8 illustrates an aortic pressure waveform and a peripheral pulse pressure waveform together with the left ventricular pressure waveform. The left ventricular pressure waveform is illustrated by a solid line. The aortic pressure waveform and the peripheral pulse pressure waveform are illustrated as a one-dot chain line and a dashed line, respectively. The peripheral pulse pressure waveform is a waveform of pulse pressure observed in an arm, a wrist, or the like of the human body 20. The aortic pressure waveform is acquired by an invasive method. The peripheral pulse pressure waveform can be acquired by a non-invasive method, for example, a non-invasive continuous blood pressure measurement method using a tonometer.

Parameters related to the arterial pressure such as a diastolic blood pressure, a systolic blood pressure, the maximum velocity of a pulse pressure waveform rise, a blood pressure value difference between a rise start point of the peripheral pulse pressure waveform and a dicrotic notch, and a pulse wave augmentation index (AI) can be acquired or calculated from the peripheral pulse pressure waveform.

The diastolic blood pressure is the pressure applied to a blood vessel when the heart is expanded. The diastolic blood pressure is also called the lowest blood pressure. The systolic blood pressure is the pressure applied to a blood vessel when the heart contracts. The systolic blood pressure is also called the maximum blood pressure.

The maximum velocity of the pulse pressure waveform rise is the maximum value of a slope dP/dt of the peripheral pulse pressure waveform due to the contraction of the heart.

The blood pressure value difference between the rise start point of the peripheral pulse pressure waveform and the dicrotic notch indicates a difference in blood pressure value between the diastolic blood pressure and the dicrotic notch as illustrated in Fig. 8. The dicrotic notch is an inflection point of the peripheral pulse pressure waveform caused by vibration due to the aortic valve closing.

The pulse wave augmentation index is a proportion of a reflected-wave component to the blood pressure, and can be acquired by analyzing the peripheral pulse pressure waveform.

Further, parameters related to the heartbeat such as a heart rate, isovolumetric systolic time, a pulse wave velocity, and systolic time can be acquired or calculated from outputs of the electrocardiogram, the microphone, and the like and the peripheral pulse pressure waveform.

The heart rate can be measured by the electrocardiogram.

The isovolumetric systolic time means a time period in which the mitral and aortic valves are closed. During the Isovolumetric systolic time, the volume of the left ventricle does not change and the left ventricular pressure rises. The isovolumetric systolic time is measured as the time from a Q-wave measured by the electrocardiogram to an I-sound of the heart sound acquired by the microphone. The I-sound is a sound of the mitral valve closing.

The pulse wave velocity is a velocity at which a pulse wave, which is a wave of pressure of blood pumped out of the heart, travels through an artery to a peripheral blood vessel. The pulse wave velocity is measured as the time from a Q-wave measured by the electrocardiogram to the rise start point of the peripheral pulse pressure waveform.

The systolic time is time of systole in which the ventricle contracts. The systole includes isovolumetric systole and an ejection phase. The ejection phase is a period in which the aortic valve opens and blood in the left ventricle is pushed out to the aorta. The systolic time is measured as the time from the rise start point of the peripheral pulse pressure waveform to the dicrotic notch using the peripheral pulse pressure waveform.

The above parameters are examples. The ventricular pressure waveform estimation device 10 may use another parameter as an explanatory variable. For the estimation of the left ventricular pressure waveform or the right ventricular pressure waveform, the ventricular pressure waveform estimation device 10 can use one or more parameters selected from the above-described parameters. The left ventricular pressure waveform and the right ventricular pressure waveform can be estimated using the same parameters.

(Estimation of Ventricular Pressure Waveform)

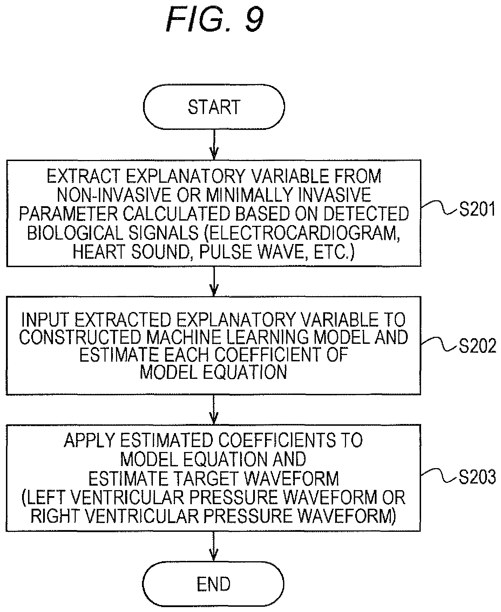

A procedure in which the calculation unit 12 of the ventricular pressure waveform estimation device 10 estimates the left ventricular pressure waveform or the right ventricular pressure waveform will be described with reference to a flowchart of Fig. 9. The flowchart of Fig. 9 illustrates a procedure of a ventricular pressure waveform estimation method executed by the ventricular pressure waveform estimation device 10.

First, the calculation unit 12 extracts explanatory variables for calculating the coefficients a, b, c, and k from non-invasive parameters or minimally invasive parameters calculated based on biological signals detected by the first detection unit 15 and the second detection unit 16 (step S201). The biological signals are the electrocardiogram, the heart sound, the pulse wave, and the like. The non-invasive parameters or the minimally invasive parameters may be calculated by the calculation unit 12 based on the biological signals detected by the first detection unit 15 and the second detection unit 16. The explanatory variables are the parameters X, Y, and Z of each of the machine learning models N (N = 1 to 4).

Next, the calculation unit 12 inputs the extracted explanatory variables to each of the constructed machine learning models N (N = 1 to 4), and estimates values of the coefficients a, b, c, and k of the model equation (step S202).

The calculation unit 12 applies the values of the coefficients a, b, c, and k estimated in step S202 to Formula (1), which is the model equation, and estimates a target left ventricular pressure waveform or right ventricular pressure waveform (step S203).

As described above, the calculation unit 12 can estimate the left ventricular pressure waveform or the right ventricular pressure waveform of the patient based on the biological signal detected in a non-invasive or minimally invasive manner using the machine learning model. Therefore, a waveform shape of the left ventricular pressure or the right ventricular pressure can be estimated by a non-invasive or minimally invasive method according to the ventricular pressure waveform estimation device 10 of the present disclosure. As a result, a medical worker such as a doctor can grasp a sign of a change in a symptom of the patient with heart failure at an early stage by a simple method, and thus, it is possible to help prevent a deterioration of the symptom by prescribing a medicine to the patient early.

Furthermore, the calculation unit 12 can calculate a value of LVEDP or PCWP from the estimated left ventricular pressure waveform or right ventricular pressure waveform. The LVEDP corresponds to the pressure at a rise time (that is, when the time is 0 ms) of the estimated left ventricular pressure waveform. The PCWP can be calculated as the pressure at a predetermined or arbitrary time from the estimated right ventricular pressure waveform. For example, the lowest value, the highest value, an average value, and the like of the PCWP can be acquired or calculated from the estimated right ventricular pressure waveform.

The calculation unit 12 can further obtain an index of contractility of the left heart or the right heart from a slope (dP/dt) of the estimated left ventricular pressure waveform or right ventricular pressure waveform. It can be determined that the contractility is high if the slope of the left ventricular pressure waveform or the right ventricular pressure waveform is large, and that the contractility is low if the slope is small. As a result, the ventricular pressure waveform estimation device 10 can quickly grasp a deterioration in a systolic function of the heart of the patient in a non-invasive or minimally invasive manner.

In the above embodiment, it is assumed that a change in the ventricular pressure can be expressed by the model equation of Formula (1) according to the two-element model of the Windkessel model. In addition, it is assumed that the coefficients of the model equation are four coefficients of a, b, c, and k. In other embodiments, different model equations may be employed based on different models. The different models may include a three-element model and a four-element model of the Windkessel model. Based on the different models, the number of coefficients in the model equations may also be numbers other than four.

(Pulmonary Artery Pressure Waveform Estimation Device)

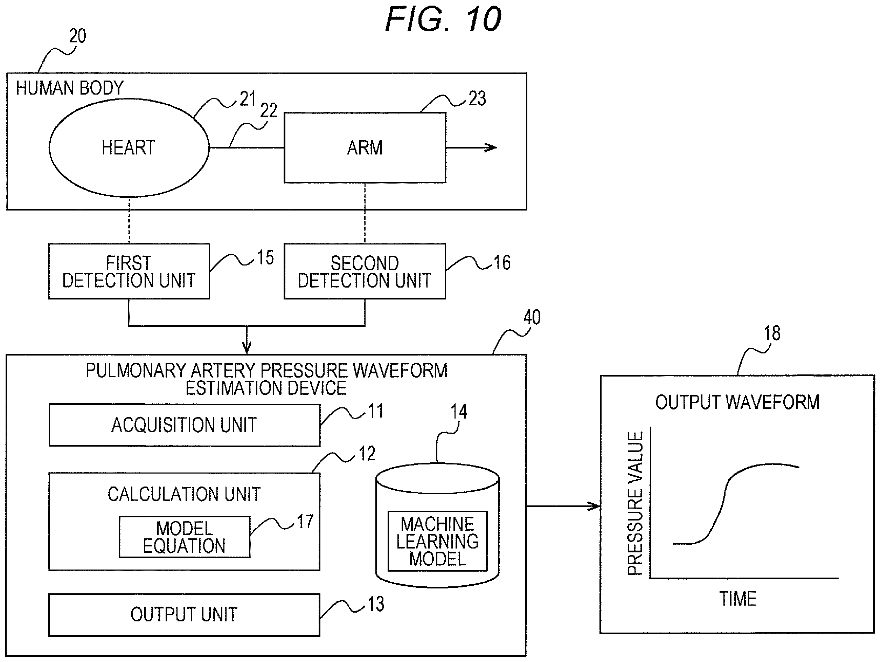

In the ventricular pressure waveform estimation device 10 of the above embodiment, at least a part of the left ventricular pressure waveform or the right ventricular pressure waveform is estimated by estimating each of the plurality of coefficients of the model equation including the coefficients and indicating the left ventricular pressure waveform or the right ventricular pressure waveform by using values of the one or more parameters related to the heartbeat or arterial pressure. According to the same method, it is possible to provide a pulmonary artery pressure waveform estimation device that estimates at least a part of a pulmonary artery pressure waveform by estimating each of a plurality of coefficients of a model equation including the coefficients and indicating the pulmonary artery pressure waveform using values of one or more parameters related to a heartbeat or an arterial pressure. Therefore, in Fig. 1, the "ventricular pressure waveform estimation device" can be replaced with the "pulmonary artery pressure waveform estimation device". Fig. 10 illustrates a configuration of a device including a pulmonary artery pressure waveform estimation device 40 according to an embodiment of the present disclosure. Since each of components in Fig. 10 is common to each of components in Fig. 1, the same reference signs as those of the components in Fig. 1 are given, and the description of those components is omitted.

Similarly to the ventricular pressure waveform estimation device 10 of Fig. 1, the pulmonary artery pressure waveform estimation device 40 estimates each coefficient of the plurality of coefficients of the model equation by using the one or more parameters as explanatory variables of a machine learning model. As the model equation, the same formula as Formula (1) can be used. The learning model of machine learning can be constructed according to a flowchart in which the "left ventricular pressure waveform or right ventricular waveform" in step S101 is replaced with the "pulmonary artery pressure waveform" in the flowchart illustrated in Fig. 4.

Similar to the above embodiment, the one or more parameters may include one or more parameters related to the heartbeat and one or more parameters related to the arterial pressure. The one or more parameters may include at least any of a diastolic blood pressure, a systolic blood pressure, a maximum velocity of a pulse pressure waveform rise, a blood pressure value difference between a rise start point of a peripheral pulse pressure waveform and a dicrotic notch, a pulse wave augmentation index pulse wave augmentation index, a heart rate, an isovolumetric systolic time, a pulse wave velocity, and a systolic time. The calculation unit 12 of the pulmonary artery pressure waveform estimation device 40 may calculate a value of pulmonary artery pressure and/or a value of pulmonary capillary wedge pressure (PCWP) based on at least a part of the pulmonary artery pressure waveform. The calculation unit 12 of the pulmonary artery pressure waveform estimation device 40 may estimate a value of left ventricular end-diastolic pressure (LVEDP) based on at least a part of the pulmonary artery pressure waveform.

Similar to the ventricular pressure waveform estimation device 10, the pulmonary artery pressure waveform estimation device 40 may include the first detection unit 15 that detects one or more first biological signals related to the heartbeat and one or more second detection units 16 that detect one or more second biological signals related to the arterial pressure. A value of the parameter may be determined based on at least any of the first biological signal and the second biological signal. The first detection unit 15 and the second detection unit 16 may detect the first biological signal and the second biological signal, respectively, by a non-invasive or minimally invasive method.

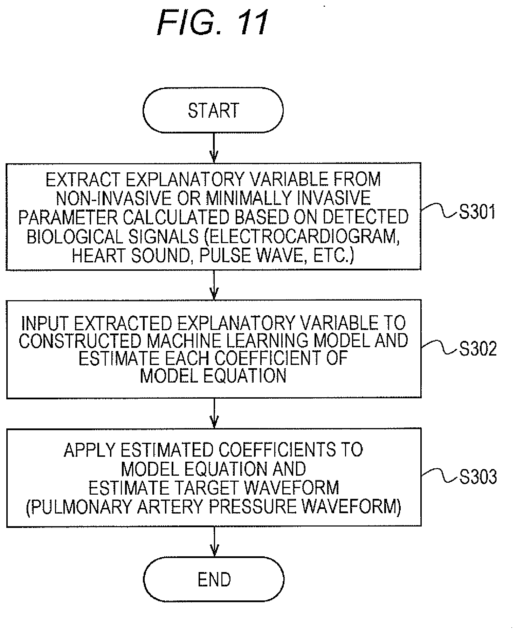

Fig. 11 is a flowchart illustrating a procedure for estimating the pulmonary artery pressure waveform. The flowchart of Fig. 11 is obtained by replacing the "left ventricular pressure waveform or right ventricular pressure waveform" in step S203 in the flowchart of Fig. 9 with the "pulmonary artery pressure waveform" in step S303. The other points are the same as those in the flowchart of Fig. 9, and thus, the description of those points is omitted.

Although the embodiments according to the present disclosure have been described based on the drawings and examples, it should be noted that those skilled in the art can easily make various modifications or changes based on the present disclosure. Therefore, it should be noted that these modifications or alterations fall within the scope of the present disclosure. For example, functions and the like included in each component, each step, or the like can be rearranged so as not to be logically inconsistent, and a plurality of components, steps, and the like can be combined into one or divided. Although the embodiments according to the present disclosure have been described mainly with respect to the devices, the embodiments according to the present disclosure can also be realized as methods including steps executed by each of the components of the devices, respectively. The embodiments according to the present disclosure can also be realized as methods or programs executed by processors included in the devices or storage media recording the programs, respectively. It should be understood that these are also included in the scope of the present disclosure.

Industrial Applicability

The ventricular pressure waveform estimation device, the ventricular pressure waveform estimation method, and the ventricular pressure waveform estimation program of the present disclosure can be applied to a monitoring device or the like for grasping a disease state of a patient with heart failure or the like, assessing a cardiac function, and the like.

Reference Signs List

10 ventricular pressure waveform estimation device
11 acquisition unit
12 calculation unit
13 output unit
14 storage unit
15 first detection unit
16 second detection unit
17 model equation
18 output waveform
20 human body
21 heart
22 blood vessel
23 arm
30 left ventricular pressure waveform
31 partial waveform
40 pulmonary artery pressure waveform estimation device

**Claims**

1. A ventricular pressure waveform estimation device comprising a calculation unit configured to estimate a plurality of coefficients of a model equation, the model equation configured to indicate a left ventricular pressure waveform or a right ventricular pressure waveform by using values of one or more parameters related to a heartbeat or an arterial pressure to estimate at least a part of the left ventricular pressure waveform or the right ventricular pressure waveform.

2. The ventricular pressure waveform estimation device according to claim 1, wherein the calculation unit is configured to estimate each coefficient of the plurality of coefficients of the model equation by using the one or more parameters as explanatory variables of a machine learning model.

3. The ventricular pressure waveform estimation device according to claim 2, wherein the machine learning model is constructed by acquiring a measured waveform of at least a part of the left ventricular pressure waveform or the right ventricular pressure waveform, calculating values of the plurality of coefficients by fitting the model equation to the measured waveform, generating a data set for machine learning in which the one or more parameters in the acquisition of the measured waveform are used as explanatory variables and the plurality of coefficients are used as objective variables, respectively, and executing machine learning using the data set for machine learning.

4. The ventricular pressure waveform estimation device according to any one of claims 1 to 3, wherein the one or more parameters include one or more parameters related to the heartbeat and one or more parameters related to the arterial pressure.

5. The ventricular pressure waveform estimation device according to any one of claims 1 to 4, wherein the model equation is

[Math. 1]

$$P(t) = \frac{a}{1 + be^{-kt}} + c$$

where a, b, c, and k are the coefficients.

6. The ventricular pressure waveform estimation device according to any one of claims 1 to 5, wherein the one or more parameters include at least any of a diastolic blood pressure, a systolic blood pressure, a maximum velocity of a pulse pressure waveform rise, a blood pressure value difference between a rise start point of a peripheral pulse pressure waveform and a dicrotic notch, a pulse wave augmentation index pulse wave augmentation index, a heart rate, an isovolumetric systolic time, a pulse wave velocity, and a systolic time.

7. The ventricular pressure waveform estimation device according to any one of claims 1 to 6, wherein the calculation unit is configured to calculate a value of left ventricular end-diastolic pressure (LVEDP) based on at least a part of the estimated left ventricular pressure waveform.

8. The ventricular pressure waveform estimation device according to any one of claims 1 to 6, wherein the calculation unit is configured to calculate a value of pulmonary capillary wedge pressure (PCWP) based on at least a part of the estimated right ventricular pressure waveform.

9. The ventricular pressure waveform estimation device according to any one of claims 1 to 8, wherein the calculation unit is configured to calculate an index of contractility of a left ventricle or a right ventricle based on a slope of the estimated left ventricular pressure waveform or the estimated right ventricular pressure waveform.

10. The ventricular pressure waveform estimation device according to any one of claims 1 to 9, comprising: a first detection unit configured to detect one or more first biological signals related to the heartbeat; and one or more second detection units configured to detect one or more second biological signals related to the arterial pressure, and wherein the values of the one or more parameters is determined based on one or more of the one or more first biological signal and the one or more second biological signal.

11. The ventricular pressure waveform estimation device according to claim 10, wherein the first detection unit and the second detection unit detect the first biological signal and the second biological signal, respectively, by a non-invasive method.

12. The ventricular pressure waveform estimation device according to claim 10, wherein the first detection unit and the second detection unit detect the first biological signal and the second biological signal, respectively, by a non-invasive or minimally invasive method.

13. A ventricular pressure waveform estimation method for estimating at least a part of a left ventricular pressure waveform or a right ventricular pressure waveform by a computer, the method comprising
estimating each of a plurality of coefficients of a model equation including the coefficients and indicating a left ventricular pressure waveform or a right ventricular pressure waveform by using values of one or more parameters related to a heartbeat or an arterial pressure to estimate at least a part of the left ventricular pressure waveform or the right ventricular pressure waveform.

14. A ventricular pressure waveform estimation program causing a computer to execute processing of estimating each of a plurality of coefficients of a model equation including the coefficients and indicating a left ventricular pressure waveform or a right ventricular pressure waveform by using values of one or more parameters related to a heartbeat or an arterial pressure to estimate at least a part of the left ventricular pressure waveform or the right ventricular pressure waveform.

15. A pulmonary artery pressure waveform estimation device comprising a calculation unit that estimates each of a plurality of coefficients of a model equation including the coefficients and indicating a pulmonary artery pressure waveform by using values of one or more parameters related to a heartbeat or an arterial pressure to estimate at least a part of the pulmonary artery pressure waveform.

16. The pulmonary artery pressure waveform estimation device according to claim 15, wherein the calculation unit estimates each coefficient of the plurality of coefficients of the model equation by using the one or more parameters as explanatory variables of a machine learning model.

17. The pulmonary artery pressure waveform estimation device according to claim 16, wherein the machine learning model is constructed by acquiring a measured waveform of at least a part of the pulmonary artery pressure waveform, calculating values of the plurality of coefficients by fitting the model equation to the measured waveform, generating a data set for machine learning in which the one or more parameters in the acquisition of the measured waveform are used as explanatory variables and the plurality of coefficients are used as objective variables, respectively, and executing machine learning using the data set for machine learning.

18. The pulmonary artery pressure waveform estimation device according to any one of claims 15 to 17, wherein the one or more parameters include one or more parameters related to a heartbeat and one or more parameters related to arterial pressure.

19. The pulmonary artery pressure waveform estimation device according to any one of claims 15 to 18, wherein the model equation is

[Math. 2]

$$P(t) = \frac{a}{1 + be^{-kt}} + c$$

where a, b, c, and k are the coefficients.

20. The pulmonary artery pressure waveform estimation device according to any one of claims 15 to 19, wherein the one or more parameters include at least any of a diastolic blood pressure, a systolic blood pressure, a maximum velocity of a pulse pressure waveform rise, a blood pressure value difference between a rise start point of a peripheral pulse pressure waveform and a dicrotic notch, a pulse wave augmentation index pulse wave augmentation index, a heart rate, an isovolumetric systolic time, a pulse wave velocity, and a systolic time.

21. The pulmonary artery pressure waveform estimation device according to any one of claims 15 to 20, wherein the calculation unit is configured to be capable of calculating a value of pulmonary artery pressure based on at least a part of the estimated pulmonary artery pressure waveform.

22. The pulmonary artery pressure waveform estimation device according to any one of claims 15 to 20, wherein the calculation unit is configured to be capable of estimating a value of left ventricular end-diastolic pressure (LVEDP) based on at least a part of the estimated pulmonary artery pressure waveform.

23. The pulmonary artery pressure waveform estimation device according to any one of claims 15 to 20, wherein the calculation unit is configured to be capable of calculating a value of pulmonary capillary wedge pressure (PCWP) based on at least a part of the estimated pulmonary artery pressure waveform.

24. The pulmonary artery pressure waveform estimation device according to any one of claims 15 to 23, wherein the calculation unit is configured to be capable of calculating an index of contractility of a left ventricle or a right ventricle based on a slope of the estimated pulmonary artery pressure waveform.

25. The pulmonary artery pressure waveform estimation device according to any one of claims 15 to 24, comprising: a first detection unit that detects one or more first biological signals related to a heartbeat; and one or more second detection units that detect one or more second biological signals related to arterial pressure, wherein the value of the parameter is determined based on at least any of the first biological signal and the second biological signal.

26. The pulmonary artery pressure waveform estimation device according to claim 25, wherein the first detection unit and the second detection unit detect the first biological signal and the second biological signal, respectively, by a non-invasive method.

27. The pulmonary artery pressure waveform estimation device according to claim 25, wherein the first detection unit and the second detection unit detect the first biological signal and the second biological signal, respectively, by a non-invasive or minimally invasive method.

FIG. 1

*FIG. 2*

# FIG. 3

# FIG. 4

START

ACQUIRE MEASURED WAVEFORM
(LEFT VENTRICULAR PRESSURE WAVEFORM OR
RIGHT VENTRICULAR PRESSURE WAVEFORM) AND
NON-INVASIVE OR MINIMALLY INVASIVE PARAMETER
CALCULATED FROM BIOLOGICAL SIGNAL DETECTED
SIMULTANEOUSLY WITH MEASURED WAVEFORM — S101

FIT MODEL EQUATION TO MEASURED WAVEFORM
TO CALCULATE VALUES OF COEFFICIENTS OF
MODEL EQUATION — S102

GENERATE MACHINE LEARNING DATA SET HAVING
ACQUIRED PARAMETER AS EXPLANATORY VARIABLE AND
EACH COEFFICIENT OF MODEL EQUATION AS
OBJECTIVE VARIABLE — S103

EXECUTE MACHINE LEARNING AND
CONSTRUCT MACHINE LEARNING MODEL FOR
EACH COEFFICIENT — S104

END

## FIG. 5

# FIG. 6

MEASURED WAVEFORM

WAVEFORM BASED ON
MODEL EQUATION

PRESSURE VALUE [mmHg]

TIME [ms]

## FIG. 7

| PARAMETER X | PARAMETER Y | PARAMETER Z |
|---|---|---|

MACHINE LEARNING
MODEL N
(N = 1−4)

COEFFICIENTS
(a, b, c, k)

*FIG. 8*

EP 4 356 826 A1

# FIG. 9

```
                    ┌─────────────┐
                    │    START    │
                    └──────┬──────┘
                           │
                           ▼
┌──────────────────────────────────────────────┐
│   EXTRACT EXPLANATORY VARIABLE FROM            │
│   NON-INVASIVE OR MINIMALLY INVASIVE           │
│   PARAMETER CALCULATED BASED ON DETECTED       │  ⌐S201
│   BIOLOGICAL SIGNALS (ELECTROCARDIOGRAM,       │
│   HEART SOUND, PULSE WAVE, ETC.)               │
└──────────────────────┬───────────────────────┘
                       │
                       ▼
┌──────────────────────────────────────────────┐
│   INPUT EXTRACTED EXPLANATORY VARIABLE TO      │
│   CONSTRUCTED MACHINE LEARNING MODEL AND       │  ⌐S202
│   ESTIMATE EACH COEFFICIENT OF                 │
│   MODEL EQUATION                               │
└──────────────────────┬───────────────────────┘
                       │
                       ▼
┌──────────────────────────────────────────────┐
│   APPLY ESTIMATED COEFFICIENTS TO              │
│   MODEL EQUATION AND                           │
│   ESTIMATE TARGET WAVEFORM                     │  ⌐S203
│   (LEFT VENTRICULAR PRESSURE WAVEFORM OR       │
│   RIGHT VENTRICULAR PRESSURE WAVEFORM)         │
└──────────────────────┬───────────────────────┘
                       │
                       ▼
                ┌─────────────┐
                │     END     │
                └─────────────┘
```

## FIG. 10

HUMAN BODY — 20

HEART — 21

22

ARM — 23

FIRST DETECTION UNIT — 15

SECOND DETECTION UNIT — 16

PULMONARY ARTERY PRESSURE WAVEFORM ESTIMATION DEVICE — 40

ACQUISITION UNIT — 11

CALCULATION UNIT — 12

MODEL EQUATION — 17

MACHINE LEARNING MODEL — 14

OUTPUT UNIT — 13

OUTPUT WAVEFORM — 18

PRESSURE VALUE

TIME

# FIG. 11

```
        ┌─────────────┐
        │    START    │
        └──────┬──────┘
               │
               ▼
┌──────────────────────────────────────┐
│ EXTRACT EXPLANATORY VARIABLE FROM     │
│ NON-INVASIVE OR MINIMALLY INVASIVE    │
│ PARAMETER CALCULATED BASED ON DETECTED│ ～S301
│ BIOLOGICAL SIGNALS (ELECTROCARDIOGRAM,│
│ HEART SOUND, PULSE WAVE, ETC.)        │
└──────────────────┬───────────────────┘
                   │
                   ▼
┌──────────────────────────────────────┐
│ INPUT EXTRACTED EXPLANATORY VARIABLE TO│
│ CONSTRUCTED MACHINE LEARNING MODEL AND │ ～S302
│ ESTIMATE EACH COEFFICIENT OF           │
│ MODEL EQUATION                         │
└──────────────────┬───────────────────┘
                   │
                   ▼
┌──────────────────────────────────────┐
│ APPLY ESTIMATED COEFFICIENTS TO       │
│ MODEL EQUATION AND                    │ ～S303
│ ESTIMATE TARGET WAVEFORM              │
│ (PULMONARY ARTERY PRESSURE WAVEFORM)  │
└──────────────────┬───────────────────┘
                   │
                   ▼
           ┌─────────────┐
           │     END     │
           └─────────────┘
```

## INTERNATIONAL SEARCH REPORT

| International application No. |
| --- |
| **PCT/JP2022/024216** |

**A. CLASSIFICATION OF SUBJECT MATTER**

*A61B 5/021*(2006.01)i
FI:   A61B5/021

According to International Patent Classification (IPC) or to both national classification and IPC

**B. FIELDS SEARCHED**

Minimum documentation searched (classification system followed by classification symbols)

A61B5/02-5/03

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | US 2015/0265163 A1 (MARMOR, Alon) 24 September 2015 (2015-09-24) paragraphs [0046]-[0215], fig. 1-8 | 1-27 |
| A | JP 2005-517469 A (MCINTYRE, Kevin M.) 16 June 2005 (2005-06-16) paragraphs [0009]-[0036], fig. 1-3 | 1-27 |
| A | JP 2002-513309 A (KINETIC CONCEPTS, INC.) 08 May 2002 (2002-05-08) entire text, fig. 1-4 | 1-27 |
| A | JP 2016-67490 A (FUKUDA DENSHI KK) 09 May 2016 (2016-05-09) paragraph [0074], fig. 13 | 1-27 |
| A | CN 103315719 A (SHANDONG MEASUREMENT SCIENCE RESEARCH INSTITUTE) 25 September 2013 (2013-09-25) entire text, fig. 1-3 | 1-27 |

☐ Further documents are listed in the continuation of Box C.　　☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **16 August 2022** | **30 August 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2022/024216**

| Patent document cited in search report | | | Publication date (day/month/year) | Patent family member(s) | | | Publication date (day/month/year) |
|---|---|---|---|---|---|---|---|
| US | 2015/0265163 | A1 | 24 September 2015 | US | 2014/0107505 | A1 | |
| | | | | WO | 2014/057489 | A1 | |
| | | | | EP | 2906110 | A1 | |
| JP | 2005-517469 | A | 16 June 2005 | US | 2003/0153837 | A1 | |
| | | | | paragraphs [0012]-[0039], fig. 1-3 | | | |
| | | | | WO | 2003/068063 | A1 | |
| | | | | EP | 1474037 | A1 | |
| JP | 2002-513309 | A | 08 May 2002 | US | 6113548 | A | |
| | | | | entire text, fig. 1-4 | | | |
| | | | | WO | 1998/034535 | A1 | |
| | | | | EP | 1017310 | A1 | |
| JP | 2016-67490 | A | 09 May 2016 | (Family: none) | | | |
| CN | 103315719 | A | 25 September 2013 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150265163 A